(19) Europäisches Patentamt / European Patent Office / Office européen des brevets

(11) **EP 4 714 473 A1**

(12) **EUROPÄISCHE PATENTANMELDUNG**

(43) Veröffentlichungstag:
25.03.2026 Patentblatt 2026/13

(21) Anmeldenummer: 25203544.9

(22) Anmeldetag: 22.09.2025

(51) Internationale Patentklassifikation (IPC):
*A61M 1/00* (2006.01)    *A61F 9/007* (2006.01)
*A61M 3/02* (2006.01)    *B23K 26/0622* (2014.01)
*B23K 26/382* (2014.01)    *A61F 9/008* (2006.01)

(52) Gemeinsame Patentklassifikation (CPC):
**A61M 1/85; A61F 9/00736; A61M 3/0254;**
**A61M 3/0279; B23K 26/0624; B23K 26/389;**
A61F 9/00827; A61F 2009/0087; A61F 2009/00887;
A61M 1/77; A61M 2202/0468; A61M 2210/0612

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **23.09.2024 DE 102024127348**

(71) Anmelder: **Silcoplast AG**
**9427 Wolfhalden (CH)**

(72) Erfinder:
• **Gaus, Philipp**
**9323 Steinach (CH)**
• **Marinkovic, Srecko**
**9322 Egnach (CH)**

(74) Vertreter: **Müller Schupfner & Partner**
**Patent- und Rechtsanwaltspartnerschaft mbB (Muc)**
**Bavariaring 11**
**80336 München (DE)**

(54) **HÜLSE, VERWENDUNG EINES ULTRAKURZPULSLASERS, VERFAHREN ZUM BEARBEITEN EINES HÜLSENROHLINGS UND VERFAHREN ZUR HERSTELLUNG EINER HÜLSE**

(57) Hülse (1), insbesondere Silikonhülse, zum Absaugen von Bruchstücken bei der operativen Behandlung von grauem Star, wobei die Hülse (1) einen operativen Bereich (2) und einen Anschlussbereich (2), der an den operativen Bereich (2) anschließt, umfasst, wobei der operative Bereich (2) zumindest abschnittsweise mit einem Laserstrahl eines Ultrakurzpulslasers, insbesondere Femtosekundenlasers, laserbearbeitet ist, sodass durch die Laserbearbeitung Material von dem operativen Bereich (2) entfernt worden ist, wobei der Laserstrahl bei der Laserbearbeitung eine Spotgröße von 2 bis 25 Mikrometern, vorzugsweise 5 bis 22 Mikrometern, besonders bevorzugt 8 bis 20 Mikrometern und eine Pulsleistung von zumindest 25 Watt, vorzugsweise zumindest 30 Watt, besonders bevorzugt zumindest 35 Watt, aufgewiesen hat.

FIG. 1

**Beschreibung**

Die Erfindung betrifft eine Hülse zum Absaugen von Bruchstücken bei der operativen Behandlung von grauem Star, eine Verwendung eines Ultrakurzpulslasers zum Erzeugen von zumindest einer Ausnehmung in einer Hülse und/oder zum Ablängen der Hülse, ein Verfahren zum Bearbeiten eines Hülsenrohlings und ein Verfahren zur Herstellung einer Hülse.

Bei der Operation des grauen Stars am Auge wird die alte Linse zerstört. Dabei erzeugte Bruchstücke werden mit Hilfe von speziell dafür angepassten Hülsen (fachsprachlich auch als "Sleeves" bezeichnet) abgesaugt. Um einem durch das Absaugen entstehenden Vakuum entgegenzuwirken, ist typischerweise zumindest eine zusätzliche Öffnung, insbesondere seitlich, an der Hülse vorgesehen, über die Flüssigkeit nachgeschoben wird. Die Hülsen müssen dabei höchsten Anforderungen genügen. Zum einen wird eine sehr präzise Anfertigung benötigt, sowohl was die generelle Geometrie der Hülsen angeht als auch betreffend die Geometrie der Löcher der Hülse. Durch einen Rohrdurchmesser der Hülse wird ein Fluidfluss durch die Hülse bestimmt. Ebenso ist eine exakte Geometrie der Öffnung sehr wichtig. Zum anderen müssen die Hülsen eine hohe Reinheit aufweisen, was bereits im Herstellungsverfahren zu berücksichtigen ist.

Nach Fertigstellung der Hülsen werden diese typischerweise mit entsprechenden Öffnungen versehen und die Hülsen werden typischerweise noch auf ein Fertigmaß abgelängt. Sowohl die Öffnungen als auch das Ablängen können durch Stanzen ermöglicht werden. Nachteilig dabei ist aber, dass für jede benötigte Geometrie ein spezifischer Stanzeinsatz und eine Matrize eigens hergestellt werden müssen. Weiterhin hat sich gezeigt, dass eine Abnutzung der Stanzkanten durch die abrasiven Eigenschaften des verwendeten Silikonmaterials sehr hoch ist. Es wäre daher wünschenswert, eine alternative Möglichkeit zum Schaffen der Löcher und/oder zum Ablängen zu finden.

Ein Ansatz wäre es, die Öffnungen bereits während eines Spritzgießprozesses der Hülse zu erzeugen. Hierbei hat es sich jedoch ergeben, dass unerwünschte Grate entstehen, die dann in einem zusätzlichen Verfahrensschritt entfernt werden müssen. Dabei kann es zu Verunreinigungen der Hülsen kommen. Ein Ablängen der Hülsen kann im Spritzgießprozess zudem nicht optimal abgebildet werden. Ein weiterer Ansatz wäre ein Einsatz eines Laserstrahls eines herkömmlichen Lasers um die Löcher zu erzeugen und um die Hülsen abzulängen. Es hat sich jedoch gezeigt, dass der dabei entstehende Laserabbrand aus medizinischer Sicht inakzeptabel zu sein scheint. Durch den Abbrand können Rückstände und/oder sichtbare Verfärbungen an der Hülse entstehen. Damit wird ein zusätzliches Waschen der Hülsen notwendig, wodurch die Gefahr einer Verunreinigung besteht, und welches den Abbrand bislang zudem nicht vollends entfernen konnte.

Es ist daher die Aufgabe der vorliegenden Erfindung, eine Möglichkeit bereitzustellen, mit der entsprechende Hülsen ohne den Einsatz von Stanzwerkzeugen oder zumindest unter einem reduzierten Einsatz von Stanzwerkzeugen erzeugt werden können und wobei die medizinischen Anforderungen erfüllt werden können. Wünschenswert wäre weiterhin eine Möglichkeit, mit der eine verbesserte Präzision beim Bearbeiten, insbesondere Erzeugen von Öffnungen und Ablängen erzielt werden kann.

Diese Aufgabe wird mit einer Hülse gemäß Anspruch 1, einer Verwendung gemäß Anspruch 7, einem Verfahren gemäß Anspruch 7 und einem Verfahren gemäß Anspruch 10 gelöst. Weitere Merkmale, Vorteile und Ausführungsformen ergeben sich aus den abhängigen Ansprüchen, der Beschreibung sowie aus den Figuren.

Erfindungsgemäß ist eine Hülse, insbesondere Silikonhülse, zum Absaugen von Bruchstücken bei der operativen Behandlung von grauem Star vorgesehen. Die Hülse umfasst einen operativen Bereich und einen Anschlussbereich, der an den operativen Bereich anschließt, wobei der operative Bereich zumindest abschnittsweise mit einem Laserstrahl eines Ultrakurzpulslasers, insbesondere Femtosekundenlasers, laserbearbeitet ist, sodass durch die Laserbearbeitung Material von dem operativen Bereich entfernt worden ist, wobei der Laserstrahl bei der Laserbearbeitung eine Spotgröße von 2 bis 25 Mikrometern, vorzugsweise 5 bis 22 Mikrometern, besonders bevorzugt 8 bis 20 Mikrometern, und eine Pulsleistung von zumindest 25 Watt, vorzugsweise zumindest 30 Watt, besonders bevorzugt zumindest 35 Watt, aufweist bzw. aufgewiesen hat. Die Hülse kann auch als Sleeve bezeichnet werden. Unter einer Hülse ist im Rahmen dieser Erfindung allgemein eine Hülse zu verstehen, die zum Absaugen von Bruchstücken bei der operativen Behandlung von grauem Star vorgesehen bzw. geeignet ist. Besonders vorteilhaft kann es sein, wenn die Hülse aus einem Silikonmaterial gefertigt ist. Optional kann die Hülse aus einem transparenten Silikonmaterial gefertigt sein. Ein Silikonmaterial ist insbesondere ein Material, das zumindest teilweise aus Silikon besteht bzw. das Silikon umfasst. Alternativ kann die Hülse auch aus einem anderen Material gefertigt sein. Beispielsweise kann die Hülse aus Titan gefertigt sein. Insbesondere früher wurden entsprechende Hülsen oftmals aus Titan gefertigt. Silikon hat gegenüber Titan den Vorteil, dass aufgrund seiner geringeren Härte die Gefahr für Verletzungen verringert werden kann. Das Silikonmaterial kann beispielsweise aus Presssilikon und/oder Flüssigsilikon gefertigt sein. Die Hülse kann zumindest eine seitliche Öffnung, insbesondere Querbohrung oder Querloch, umfassen. Vorzugsweise weist die zumindest eine seitliche Öffnung abgerundete Ecken auf und/oder ist oval, insbesondere rund, ausgestaltet. Die Hülse weist einen operativen Bereich und einen Anschlussbereich auf. Insbesondere kann die zumindest eine seitliche Öffnung seitlich an dem operativen Bereich vorgesehen sein. Der operative Bereich schließt an den Anschlussbereich an. Insbesondere kann der operative Bereich in den Anschlussbereich übergehen. Vorzugsweise weist der operative Bereich einen geringeren Durchmesser auf als der Anschlussbereich. Beispielsweise kann der operative Bereich einen äußeren Querschnittsdurchmesser von 0,5 mm bis 4 mm, vorzugsweise 1 mm bis 2 mm aufweisen. Ein Verhältnis eines inneren Querschnittsdurchmessers des operativen Bereichs zu dem äußeren Querschnittsdurchmesser des operativen Bereichs kann beispielsweise 0,3 bis 0,95, vorzugsweise 0,5 bis 0,9, besonders bevorzugt 0,70 bis 0,87, betragen. Beispielsweise kann der Anschlussbereich einen äußeren Querschnittsdurchmesser von 5 mm bis 15 mm, vorzugsweise 6 mm bis 10 mm aufweisen. Ein Verhältnis eines inneren Querschnittsdurchmessers des Anschlussbereichs zu dem äußeren Querschnittsdurchmesser des Anschlussbereichs kann beispielsweise 0,2 bis 0,8, vorzugsweise 0,4 bis 0,75, besonders bevorzugt 0,5 bis 0,65, betragen. Es kann vorgesehen sein, dass ein Verhältnis der Länge des operativen Bereichs zu der Länge des Anschlussbereichs zwischen 0,5 bis 2, vorzugsweise 0,8 bis 1,2, liegt. Entsprechenden Maße können besonders vorteilhaft für den Zweck des Absaugens von Bruchstücken sein. Insbesondere kann eine besonders gute Einführbarkeit der Hülse mit guten Absaugeeigenschaften erzielt werden. Der Anschlussbereich kann dazu ausgestaltet sein, dass eine Leitung, beispielsweise ein Schlauch, an den Anschlussbereich angeschlossen werden kann. Insbesondere kann es vorgesehen sein, dass die Leitung insbesondere der Schlauch, über den Anschlussbereich gestülpt werden kann und/oder in den Anschlussbereich eingeführt, beispielsweise eingesteckt oder eingeschraubt, werden kann. Der Anschlussbereich kann innen oder außen einen gerippten Bereich zum Befestigen einer Leitung, insbesondere eines Schlauchs, aufweisen. Der Anschlussbereich kann ein Innengewinde aufweisen, beispielsweise zum Einschrauben eines Anschlussschlauchs und/oder -kabels. Der operative Bereich ist zumindest abschnittsweise mit einem Laserstrahl mit einer Spotgröße bzw. Laserspotgröße von 2 bis 25 Mikrometern, vorzugsweise 5 bis 22 Mikrometern, besonders bevorzugt 8 bis 20 Mikrometern eines Ultrakurzpulslasers, insbesondere Femtosekundenlasers, mit einer Pulsleistung von zumindest 25 Watt, vorzugsweise zumindest 30 Watt, besonders bevorzugt zumindest 35 Watt laserbearbeitet worden. Als Ultrakurzpulslaser werden insbesondere Laserstrahlquellen bezeichnet, die gepulstes Laserlicht mit Pulsdauern im Bereich von Bruchteilen von Sekunden aussenden. Ein Femtosekundenlaser sendet entsprechend Laserlicht mit Pulsdauern im Größenbereich von Femtosekunden. Im Rahmen der Erfindung wurde durch die Laserbearbeitung Material von dem operativen Bereich der Hülse entfernt. Beispielsweise kann eine durch Laserbohren oder, bevorzugt, Laserschneiden verursachte Ausnehmung vorgesehen sein. Bei einem Laserbohren wird lokal so viel Energie auf die zu bearbeitende Stelle gegeben, dass das Material an dieser Stelle aufgeschmolzen und teilweise verdampft wird. Bei einem Laserschneiden wird eine Kontur ausgeschnitten. Die Kontur kann insbesondere einem Rand der Ausnehmung entsprechen. Insbesondere kann die Ausnehmung dadurch erzeugt werden, bzw. erzeugt sein, dass der Laserstrahl die Kontur abfährt bis das Material entlang der Kontur vollständig durchtrennt ist. Das Laserschneiden kann gepulst oder in einem Continuous-Wave-Modus vorgesehen sein. (engl. "continuous wave", zu Deutsch: kontinuierliche Welle). Bei einem Continuous-Wave-Modus wird der Laserstrahl insbesondere kontinuierlich und mit im Wesentlichen konstanter Amplitude angewandt. Das Laserschneiden kann insbesondere ein Remote-Laserschneiden sein. Vorteilhafterweise kann mit den erfindungsgemäßen Parametern, insbesondere der Pulsleistung und der Spotgröße ein Aufschmelzen des Materials am Rande der Ausnehmung zumindest weitgehend verhindert werden. Zusätzlich oder alternativ kann die Hülse durch die Energie des Laserstrahls abgelängt sein. Während es bei vorherigen Versuchen regelmäßig zu dem Problem kam, dass ein zu großer Abbrand bei einer Laserbearbeitung von Silikonhülsen entstand, sodass eine Laserbearbeitung von Hülsen, die für den erfindungsgemäßen Zweck vorgesehen sind, als nachteilig und eher nicht zweckmäßig angesehen wurde, hat sich überraschenderweise gezeigt, dass mit der anspruchsgemäß gegebenen Laserkonfiguration ein Abbrand soweit verhindert werden kann, dass benötigte hygienische Voraussetzungen erzielt werden können. Durch die einerseits geringe Spotgröße bei andererseits hoher Energie kann die Energie des Laserstrahls so gebündelt werden und mit ausreichend kurzer Pulsdauer durchgeführt werden, sodass eine Umgebung des bearbeiteten Abschnitts der Hülse nur unwesentlich beeinflusst wird. Insbesondere ergibt sich auch eine nur geringe Wärmeeinflusszone und wenig Schmelze bzw. wenig seitlicher Schwelbrand, sodass um den bearbeiteten Bereich herum kaum wieder erstarrtes Material anfällt. Auch können Mikrorisse besser verhindert werden und die Oberflächenqualität der Hülse, insbesondere im Bereich der Bearbeitung durch die Laserstrahl, kann deutlich verbessert werden. Durch die Bearbeitung mit einem erfindungsgemäßen Laserstrahl kann sich die gefertigte Hülse insbesondere durch einen fehlenden oder nur minimal geringfügig vorhandenen Abbrand bei gleichzeitig hoher Präzision des bearbeiteten Bereichs auszeichnen. Der fehlende bzw. sehr geringfügige Abbrand kann in der fertigen Hülse durch nicht vorhandene Rückstände, zum Beispiel chemische Rückstände, nicht vorhandene Rauchspuren, fehlende sichtbare Verfärbungen erkennbar sein. Insbesondere tauchen auch keine ungewünschten Grate auf. Eine erfindungsgemäße Hülse kann besonders kostengünstig, insbesondere verglichen mit einem Stanzen von Löchern oder einem Ablängen durch Stanzen, herstellbar sein. Auch können mit relativ geringem Aufwand verschiedene Versionen von Hülsen, insbesondere mit verschiedenen Ausnehmungen, betreffend die Geometrie der Ausnehmungen, hergestellt werden.

Vorteilhafterweise umfasst der operative Bereich ein Loch bzw. eine Bohrung, insbesondere seitliches Loch bzw. seitliche Bohrung, das/die während der Laserbearbeitung durch Laserschneiden mit dem Laserstrahl erzeugt worden ist. Alternativ oder zusätzlich ist ein Ende des operativen Bereichs während der Laserbearbeitung mit dem Laserstrahl abgelängt worden. Vorteilhafterweise kann eine geometrische Ausgestaltung der Bohrung bzw. des Lochs besonders leicht anpassbar sein, beispielsweise gemäß nutzungsspezifischen Anforderungen. Es hat sich gezeigt, dass mit dem erfindungsgemäßen Lasertyp, d.h. insbesondere mit der erfindungsgemäßen Spotgröße und Pulsleistung, eine seitliche Bohrung/Loch und/oder ein Ablängen besonders effizient, präzise und zumindest weitgehend rückstandsfrei bereitgestellt werden kann. Mit der erfindungsgemäßen Laserbearbeitung kann insbesondere ein Durchmesser der Bohrung bzw. des Lochs besonders präzise angepasst werden. Die Bohrung bzw. das Loch kann vorzugsweise einen ovalen, optional runden, Querschnitt und/oder abgerundete Ecken aufweisen.

Vorteilhafterweise ist ein Laserstrahl mit einer Wellenlänge von 200 bis 500 nm, vorzugsweise 300 bis 400 nm, ganz bevorzugt 330 bis 380 nm, verwendet worden. Eine Wellenlänge im Bereich von 300 bis 400 nm hat sich als besonders günstig herausgestellt, um Hülsen üblicherweise verwendeter Silikonmaterialien besonders effizient bearbeiten zu können. Eine Wellenlänge von 330 bis 380 nm kann dabei besonders präzise Ergebnisse ermöglichen. Mit einer Wellenlänge in den genannten Bereichen ist eine Fokussierung zu einem relativ kleinen Laserspot möglich. Mit einem kleinen Laserspot kann die Laserenergie auf einen besonders kleinen Brennfleck konzentriert werden, womit es insbesondere ermöglicht wird, feine Konturen zu bearbeiten. Der genannte Wellenlängenbereich von 330 bis 380 nm ist besonders günstig, da, hiermit einerseits ein besonders präzises Arbeiten möglich ist, wobei gleichzeitig eine Bereitstellung einer solchen Wellenlänge (gegenüber noch geringeren Wellenlängen) noch relativ gut möglich ist.

Vorteilhafterweise ist ein Modul zur Frequenzvervielfachung verwendet worden, um die Wellenlänge des Laserstrahls einzustellen. Durch die Verwendung eines Moduls zur Frequenzvervielfachung kann vorteilhafter Weise ein Laser mit nativ höherer Wellenlänge verwendet werden, was die Verfügbarkeit von geeigneten Lasern deutlich verbessern kann, während gleichzeitig eine geeignet kurze Wellenlänge auf das Hülsenmaterial einwirken kann. Eine Frequenzverdreifachung kann auch kurz als **THG** (third harmonic generation) bezeichnet werden.

Vorteilhafterweise ist der verwendete Ultrakurzpulslaser dazu ausgestaltet, Laserlicht im Infrarotbereich, insbesondere mit einer Wellenlänge von zumindest 900 nm, vorzugsweise zwischen 900 und 1500 nm, ganz bevorzugt zwischen 1000 und 1200 nm, zu erzeugen, wobei ein Modul zur Frequenzvervielfachung, insbesondere Frequenzverdreifachung, eingesetzt wurde, um die Wellenlänge des Laserstrahls einzustellen. Eine Wellenlänge in diesem Bereich, insbesondere von über 900 nm, kann besonders gut und auch günstig hergestellt werden, weil viele industriell verwendete Leser in diesem Wellenlängenbereich operieren. Dadurch kann ein Leser mit geeigneten Eigenschaften, insbesondere einer entsprechenden Spotgröße und einer sprechenden Pulsleistung, relativ einfach und kostengünstig bereitgestellt werden, wobei gleichzeitig eine vorteilhaft niedrige Wellenlänge durch die Frequenzvervielfachung erzeugt werden kann. Besonders vorteilhaft ist dabei ein Wellenlängenbereich zwischen 1000 und 1200 nm, insbesondere in Kombination mit einer Frequenzverdreifachung, weil mit dieser Kombination mittels eines relativ gut verfügbaren Lesers eine für die Herstellung der benötigten Hülse besonders günstige Wellenlänge erzeugt werden kann. Beispielsweise kann der Ultrakurzpulslaser eine Wellenlänge in der Größenordnung von 1060 nm erzeugen und das Modul zur Frequenzvervielfachung verringert die Wellenlänge auf eine Wellenlänge im Bereich von 355 nm.

Vorteilhafterweise hat der verwendete Laserstrahl bei der Laserbearbeitung eine Pulsenergie von zumindest 120 Mikrojoule, vorzugsweise zumindest 150 Mikrojoule, besonders bevorzugt zumindest 180 Mikrojoule, aufgewiesen. Mit anderen Worten kann der operative Bereich mit einem Laserstrahl mit einer Pulsenergie von zumindest 120 Mikrojoule, vorzugsweise zumindest 150 Mikrojoule, besonders bevorzugt zumindest 180 Mikrojoule laserbearbeitet sein.

Vorteilhafterweise hat der verwendete Laserstrahl bei der Laserbearbeitung eine Beugungsmaßzahl $M^2 < 1{,}2$ aufgewiesen. Die Beugungsmaßzahl kann insbesondere die Strahlqualität $K$ zu $K = 1/M^2$ definieren. Eine theoretisch optimale Strahlqualität liegt bei $K = 1$ und $M^2 = 1$ vor. Entsprechend bedeutet eine geringere Beugungsmaßzahl $M^2$ eine größere Strahlqualität. Eine niedrigere Beugungsmaßzahl erlaubt ein besseres Fokussieren des Laserstrahls durch eine Fokussieroptik, z.B. durch eine optische Linse, und gibt ein Maß dafür, wie nahe ein Laserstrahl einem idealen Gaußstrahl ist. Die Beugungsmaßzahl kann beispielsweise gemäß ISO/DIS 11146 bestimmt werden. Es hat sich gezeigt, dass mit einer Maßzahl $M^2 < 1{,}2$ ein Abbrand besonders gut verhindert werden kann. Insbesondere kann mit einem solchen Wert eine besonders gute Fokussierbarkeit erzielt werden. Die Maßzahl kann insbesondere durch die Geometrie des Laserresonators eingestellt bzw. erreicht werden.

Vorteilhafterweise ist die Hülse so hergestellt, dass während der Bearbeitung der Hülse mit dem Laserstrahl eine Laserrauch-Absaugvorrichtung eingesetzt wurde, um einen entstehenden Laserabbrand abzusaugen. Vorzugsweise kann die Laserrauch-Absaugvorrichtung einen Absaugventilator umfassen. Der Absaugventilator kann eine Leistung von 0,5 kW bis 5 kW vorzugsweise 0,8 kW bis 2 kW aufweisen. Der Absaugventilator kann einen Luftdurchsatz von zumindest 100 m$^3$/h, vorzugsweise zumindest 200 m$^3$/h, besonders bevorzugt 200 bis 400 m$^3$/h, aufweisen. Es hat sich rausgestellt das mit dieser Leistung und diesem Luftdurchsatz eine sehr effiziente Absaugung des verbleibenden auftretenden Abbrandes ermöglicht werden kann. Insbesondere bei 0,8 kW bis 2 kW Absaugleistung und einem Luftdurchsatz von 200 bis 400 m$^3$/h kann eine nahezu rückstandsfreie Laserbearbeitung ermöglicht werden.

Vorteilhafterweise umfasst der operative Bereich einen ersten rohrartiger Hohlabschnitt mit einem seitlichen Loch bzw. einer seitlichen Bohrung, wobei das seitliche Loch bzw. die seitliche Bohrung durch Laserschneiden mit dem Laserstrahl erzeugt worden ist. Der erste rohrartige Hohlabschnitt kann insbesondere dazu ausgestaltet sein, dass durch ihn abgesaugte Bruchstücke, die insbesondere bei der Behandlung des grauen Stars anfallen, transportiert werden können und/oder das durch ihn Flüssigkeit für einen Druckausgleich nachgeschoben werden kann. Beispielsweise kann ein Verhältnis eines des Innendurchmessers ersten rohrartigen Hohlabschnitts zu einem Außendurchmesser des ersten rohrartigen Hohlabschnitts im Bereich von 0,5-0,0,99, vorzugsweise 0,6 bis 0,95, besonders bevorzugt 0,8 bis 0,90 liegen. Damit können besonders gute Absaugeigenschaften erzielbar sein.

Vorteilhafterweise umfasst der Anschlussbereich der Hülse einen zweiten rohrartigen Hohlabschnitt, wobei der zweite rohrartige Hohlabschnitt einen größeren Querschnittsdurchmesser, insbesondere äußeren Querschnittsdurchmesser, aufweist als der erste rohrartige Hohlabschnitt, insbesondere einen zumindest zweimal so großen Querschnittsdurchmesser, vorzugsweise zumindest dreimal so großen Querschnittsdurchmesser. Besonders bevorzugt kann ein innerer Querschnittsdurchmesser des zweiten rohrartigen Hohlabschnitts zu einem inneren Querschnittsdurchmesser des ersten rohrartigen Hohlabschnitts in einem Verhältnis von 0,2 bis 0,3 sein. Besonders bevorzugt kann ein äußerer Querschnittsdurchmesser des zweiten rohrartigen Hohlabschnitts zu einem äußeren Querschnittsdurchmesser des ersten rohrartigen Hohlabschnitts in einem Verhältnis von 0,1 bis 0,25 sein. Es kann vorzugsweise vorgesehen sein, dass die Achsen des ersten rohrartigen Hohlabschnitts und des zweiten rohrartigen Hohlabschnitts parallel zueinander verlaufen. Insbesondere kann der erste rohrartige Hohlabschnitt im Wesentlichen konzentrisch zu dem zweiten rohrartigen Hohlschnitt angeordnet sein. Beispielsweise kann ein Verhältnis eines Innendurchmessers des zweiten rohrartigen Hohlabschnitts zu einem Außendurchmesser des zweiten rohrartigen Hohlabschnitts im Bereich von 0,2-0,8, vorzugsweise 0,4 bis 0,75, besonders bevorzugt 0,5 bis 0,7 liegen. Damit können einerseits besonders gute Absaugeigenschaft erzielbar sein und andererseits kann ein Anschluss, insbesondere ein Anschlussschlauch, besonders gut an dem Anschlussbereich befestigbar sein.

Vorteilhafterweise ist ein Verhältnis des Außendurchmessers des ersten rohrartigen Hohlabschnitts zu einem Innendurchmesser des ersten rohrartigen Hohlabschnitts geringer als ein Verhältnis des Außendurchmessers des zweiten rohrartigen Hohlabschnitts zu einem Innendurchmesser des zweiten rohrartigen Hohlabschnitts. Insbesondere kann ein Verhältnis des Außendurchmessers des ersten rohrartigen Hohlabschnitts zu einem Innendurchmesser des ersten rohrartigen Hohlabschnitts um einen Faktor im Bereich von 0,5 bis 0,9, vorzugsweise 0,6 bis 0,8, geringer als ein Verhältnis des Außendurchmessers des zweiten rohrartigen Hohlabschnitts zu einem Innendurchmesser des zweiten rohrartigen Hohlabschnitts sein. Damit können besonders günstige Strömungseigenschaften in der Hülse erzielbar sein, insbesondere auch mit Hinblick auf den Anschluss eines Anschlussschlauchs an den Anschlussbereich.

Vorteilhafterweise weist der Anschlussbereich einen Übergangsbereich zwischen dem ersten rohrartigen Hohlabschnitt und dem zweiten rohrartigen Hohlabschnitt auf, sodass der erste rohrartige Hohlabschnitt in den zweiten rohrartigen Hohlabschnitt übergeht, wobei insbesondere ein innerer Hohlbereich des ersten rohrartigen Hohlabschnitts in einen inneren Hohlbereich des zweiten rohrartigen Hohlabschnitts übergeht. Vorzugsweise kann der Übergangsbereich im Wesentlichen konzentrisch zu dem ersten rohrartigen Hohlabschnitt und zu dem zweiten rohrartigen Hohlabschnitt ausgestaltet sein. Ein Übergangsbereich kann besonders gute Fluidflusseigenschaften und/oder ein besonders Effizientes Absaugen von Bruchstücken ermöglichen.

Vorteilhafterweise sind der Anschlussbereich und der operative Bereich ein gemeinsamer einteiliger Körper. Dies ermöglicht eine besonders einfache Herstellung, beispielsweise durch ein Spritzgießen. Weiterhin können somit eine gute Stabilität und eine gute Dichte der gesamten Hülse ermöglicht werden.

Vorteilhafterweise ist die Hülse spritzgegossen. Ein Spritzgießverfahren kann eine besonders einfache Möglichkeit sein, eine Hülse bzw. einen Hülsenrohling, der noch laserbearbeitet wird, herzustellen.

Vorteilhafterweise ist die Hülse aus einem transparenten Silikonmaterial gefertigt, insbesondere spritzgegossen. Eine Hülse aus transparentem Silikonmaterial kann besonders Vorteilhaft bei der Verwendung als Operationswerkzeug für die Behandlung des Grauen Stars sein.

Ein weiterer Aspekt der Erfindung ist die Verwendung eines Ultrakurzpulslasers, insbesondere Femtosekundenlasers, zum Entfernen von Material durch Laserbearbeitung von einem Hülsenrohling, insbesondere Silikonhülsenrohling, insbesondere Erzeugen von zumindest einer Ausnehmung, insbesondere Loch bzw. Bohrung, in dem Hülsenrohling und/oder zum Ablängen des Hülsenrohlings, bei der Herstellung einer Hülse, insbesondere Silikonhülse, zum Absaugen von Bruchstücken bei der operativen Behandlung von grauem Star, wobei der verwendete Ultrakurzpulslaser einen Laserstrahl mit einer Spotgröße von 2 bis 25 Mikrometern, vorzugsweise 5 bis 22 Mikrometern, besonders bevorzugt 8 bis 20 Mikrometern und einer Pulsleistung von zumindest 25 Watt, vorzugsweise zumindest 30 Watt, besonders bevorzugt zumindest 35 Watt, erzeugt.

Ein weiterer Aspekt der Erfindung ein Verfahren zum Bearbeiten eines Hülsenrohlings, insbesondere Silikonhülsenrohlings, zum Absaugen von Bruchstücken bei der operativen Behandlung von grauem Star, um Material von dem Hülsenrohling zu entfernen, insbesondere um den Hülsenrohling abzulängen und/oder mit zumindest einer Ausnehmung zu versehen, wobei das Verfahren umfasst:

- Bereitstellen des Hülsenrohlings
- Fokussieren eines Laserstrahls auf zumindest einen vorbestimmten Fokuspunkt auf dem Hülsenrohling, sodass von dem Hülsenrohling an dem zumindest einen Fokuspunkt mit Hilfe der Laserenergie Material entfernt wird, wobei der Hülsenrohling insbesondere mit Hilfe der Laserenergie abgelängt und/oder mit einer Ausnehmung, insbesondere Loch bzw. Bohrung, versehen wird; wobei der Laserstrahl von einem Ultrakurzpulslaser, insbesondere Femtosekundenlaser, erzeugt wird, wobei der Laserstrahl eine Spotgröße von 2 bis 25 Mikrometern, vorzugsweise 5 bis 22 Mikrometern, besonders bevorzugt 8 bis 20 Mikrometern, und eine Pulsleistung von zumindest 25 Watt, vorzugsweise zumindest 30 Watt, besonders bevorzugt zumindest 35 Watt, aufweist. Mit dem Verfahren kann insbesondere eine erfindungsgemäße Hülse aus dem Hülsenrohling hergestellt werden. Der Begriff Hülsenrohling ist im Rahmen dieser Erfindung breit zu verstehen. Er bezeichnet insbesondere allgemein eine Hülse in einem Herstellungsstadium vor der Laserbearbeitung bzw. vor dem Hinzufügen entsprechender Löcher/-Ausnehmung und/oder vor dem Ablängen. Das Entfernen von Lasermaterial kann vorzugsweise automatisiert computergesteuert erfolgen, insbesondere indem der Laserstrahl und die Positionierung des Laserstrahls von einem Computerprogramm gesteuert wird. Das Computerprogramm kann einstellbare Parameter umfassen, die entsprechend nutzungsspezifischen Anforderungen anpassbar sind. Beispielsweise kann das Computerprogramm dazu ausgestaltet sein, dass eine Geometrie einer Ausnehmung, insbesondere eines Lochs, erzeugt durch Laserschneiden, insbesondere Tiefe, Form und/oder Querschnitt, einstellbar sind. Die Ausnehmung, insbesondere Loch kann insbesondere durch ein Laserschneiden mit dem Laserstrahl erzeugt werden. Bei einem Laserschneiden fährt der Laser eine Kontur des vorgesehenen Lochs ab, bis das Material vollständig durchtrennt ist. Vorteilhafterweise kann mit den erfindungsgemäßen Parametern, insbesondere der Pulsleistung und der Spotgröße ein Aufschmelzen des Materials am Rande der Ausnehmung bzw. des Lochs zumindest weitgehend verhindert werden. Das Ablängen kann insbesondere durch eine Laserstrahlschneiden vorgesehen sein. Vorzugsweise kann für den Laserstrahl ein gepulster Betrieb vorgesehen sein, insbesondere mit einer eine Pulsfrequenz in der Größenordnung von 1 Hz bis 1 MHz. Der verwendete Ultrakurzpulslaser ist vorzugsweise ein Femtosekundenlaser. Beispielsweise kann eine Pulsdauer von 50 fs bis 800fs, vorzugsweise 100 fs bis 500 fs, besonders bevorzugt 200 bis 300 fs, verwendet werden. Vorzugsweise beträgt eine Pulsenergie des Laserstrahls zumindest 120 Mikrojoule, vorzugsweise zumindest 150 Mikrojoule, besonders bevorzugt zumindest 180 Mikrojoule. Vorteilhafterweise kann der verwendete Laserstrahl eine Wellenlänge von 200 bis 500 nm, vorzugsweise 300 bis 400 nm, ganz bevorzugt 330 bis 380 nm aufweisen. Vorzugsweise ist der verwendete Ultrakurzpulslaser derart ausgestaltet, dass der Laserstrahl, der auf den Fokuspunkt trifft, eine Beugungsmaßzahl $M^2 < 1,2$ aufweist. Vorzugsweise kann der verwendete Ultrakurzpulslaser mit einer Kühlung, insbesondere Wasserkühlung betrieben werden. Vorzugsweise wird während der Behandlung des Hülsenrohlings mit dem Laserstrahl eine Laserrauch-Absaugvorrichtung eingesetzt, um einen entstehenden Laserabbrand abzusaugen. Der verwendete Hülsenrohling ist vorzugsweise aus einem Silikonmaterial gefertigt. Mit anderen Worten kann der Hülsenrohling ein Silikonhülsenrohling sein. Der Hülsenrohling kann optional aus einem transparenten Silikonmaterial gefertigt sein. Vorzugsweise kann der Hülsenrohling spritzgegossen sein. Vorzugsweise sind Laserparameter, insbesondere umfassend eine Leistungseinstellung, eine Frequenz und/oder eine Scangeschwindigkeit, auf das Verfahren optimiert. Die Laserparameter können versuchsweise ermittelt werden und für die weitere Verwendung gespeichert werden.

Vorteilhafterweise wird ein Modul zur Frequenzvervielfachung verwendet, um die Wellenlänge des Laserstrahls einzustellen. Insbesondere kann ein Laser mit einer höheren Wellenlänge verwendet werden als die Wellenlänge des Laserstrahls, der auf den Hülsenrohling fokussier wird bzw. der auf den Hülsenrohling trifft.

Vorteilhafterweise ist der Ultrakurzpulslaser dazu ausgestaltet, Laserlicht im Infrarotbereich, insbesondere mit einer Wellenlänge von zumindest 900 nm, vorzugsweise zwischen 900 und 1500 nm, ganz bevorzugt zwischen 1000 und 1200 nm, zu erzeugen, wobei ein Modul zur Frequenzvervielfachung, insbesondere Frequenzverdreifachung, eingesetzt wird, um die Wellenlänge des Laserstrahls, der auf den zumindest einen Fokuspunkt trifft, einzustellen, insbesondere auf einen geringeren Wellenlängenwert einzustellen. Vorzugsweise kann die Wellenlänge des Laserstrahls auf eine Wellenlänge von 200 bis 500 nm, vorzugsweise 300 bis 400 nm, ganz bevorzugt 330 bis 380 nm, eingestellt werden.

Vorteilhafterweise umfasst der Ultrakurzpulslaser ein Linsensystem umfassend, insbesondere hochreine, Vollquarzlinsen. Vollquarzlinsen können besonders gut geeignet sein, um den Laserstrahl auf eine Oberfläche des Werkstücks bzw. der Hülse zu fokussieren. Dabei können mit Vollquarzlinsen geringe Energieverluste, insbesondere durch hohe Transmission und geringe Absorption, erzielt werden.

Vorteilhafterweise umfasst der Hülsenrohling zumindest einen ersten rohrartigen Hohlabschnitt, wobei der zumindest eine Fokuspunkt auf eine Seitenwand des ersten rohrartigen Hohlabschnitts gerichtet wird, wobei die Ausnehmung als seitliche Bohrung in dem rohrartigen Hohlabschnitt durch den Laserstrahl erzeugt wird.

Vorteilhafterweise umfasst die Hülse weiterhin zumindest einen zweiten rohrartigen Hohlabschnitt, wobei der zweite rohrartige Hohlabschnitt einen größeren Querschnittsdurchmesser aufweist als der erste rohrartige Hohlabschnitt, insbesondere einen zumindest zweimal so großen Querschnittsdurchmesser, vorzugsweise zumindest dreimal so großen Querschnittsdurchmesser.

Ein weiterer Aspekt der Erfindung ist ein Verfahren zur Herstellung einer Hülse, insbesondere Silikonhülse, zum Absaugen von Bruchstücken bei der operativen Behandlung von grauem Star, umfassend die folgenden Schritte:

- Fertigen eines Hülsenrohlings, insbesondere aus einem Silikonmaterial, insbesondere Spritzgießen des Hülsenrohlings in einem Spritzgießverfahren,
- Bearbeiten des spritzgegossenen Hülsenrohlings mit einem Verfahren zum Bearbeiten eines Hülsenrohlings wie hierin beschrieben um die Hülse abzulängen und/oder mit zumindest einer Ausnehmung zu versehen. Unter einer Silikonhülse ist insbesondere eine Hülse zu verstehen, die aus einem Silikonmaterial gefertigt, insbesondere spritzgegossen, ist. Das Laserbearbeiten des spritzgegossenen Hülsenrohlings kann unmittelbar an das Spritzgießen anschließen und/oder unmittelbar in den Spritzgießprozess integriert sein. Optional kann der Hülsenrohling aus einem transparenten Silikonmaterial spritzgegossen werden.

Vorteilhafterweise wird der Hülsenrohling derart spritzgegossen, dass er zumindest einen ersten rohrartigen Hohlabschnitt umfasst, wobei der zumindest eine Fokuspunkt auf eine Seitenwand des ersten rohrartigen Hohlabschnitts gerichtet wird, wobei die Ausnehmung als seitliche Bohrung in dem rohrartigen Hohlabschnitt durch den Laserstrahl erzeugt wird.

Vorteilhafterweise wird die Hülse derart spritzgegossen, dass sie weiterhin zumindest einen zweiten rohrartigen Hohlabschnitt umfasst, und dass der zweite rohrartige Hohlabschnitt einen größeren Querschnittsdurchmesser aufweist als der erste rohrartige Hohlabschnitt, insbesondere einen zumindest zweimal so großen Querschnittsdurchmesser, vorzugsweise zumindest dreimal so großen Querschnittsdurchmesser.

Vorteilhafterweise wird die Hülse derart spritzgegossen, dass weiterhin ein Übergangsbereich zwischen dem ersten rohrartigen Hohlabschnitt und dem zweiten rohrartigen Hohlabschnitt erzeugt wird, sodass der erste rohrartige Hohlabschnitt in den zweiten rohrartigen Hohlabschnitt übergeht, wobei insbesondere ein innerer Hohlbereich des ersten rohrartigen Hohlabschnitt in einen inneren Hohlbereich des zweiten rohrartigen Hohlabschnitt übergeht.

Vorteilhafterweise wird die Hülse derart spritzgegossen, dass ein Verhältnis des Durchmessers der Außenwand des ersten rohrartigen Hohlabschnitts zu einem Innendurchmesser des ersten rohrartigen Hohlabschnitts geringer ist als ein Verhältnis des Durchmessers der Außenwand des zweiten rohrartigen Hohlabschnitts zu einem Innendurchmesser des zweiten rohrartigen Hohlabschnitts.

Die Merkmale und Vorteile die hierin im Rahmen eines Aspekts der Erfindung beschrieben werden können auch auf die jeweils anderen Aspekte der Erfindung übertragen werden. Beispielsweise kann das Verfahren zur Herstellung einer Hülse auch entsprechende Merkmale und Vorteile wie die Hülse, die Verwendung und das Verfahren zum Bearbeiten eines Hülsenrohlings aufweisen. Umgekehrt können auch die erfindungsgemäße Hülse, die erfindungsgemäße Verwendung und das erfindungsgemäße Verfahren zum Bearbeiten eines Hülsenrohlings entsprechende Merkmale und Vorteile wie das Verfahren zur Herstellung einer Hülse oder wie die anderen dieser Aspekte aufweisen.

Weitere Vorteile und Merkmale der vorliegenden Erfindung ergeben sich aus der nachfolgenden Beschreibung mit Bezug auf die Figuren. Einzelne in den gezeigten Ausführungsformen offenbarten Merkmale können auch in anderen Ausführungsformen eingesetzt werden, sofern dies nicht ausdrücklich ausgeschlossen wurde. Es zeigen:

Figur 1 eine Hülse zum Absaugen von Bruchstücken bei der operativen Behandlung von grauem Star gemäß einer Ausführungsform der Erfindung;

Figur 2 den mit einem Kreis markierten Ausschnitt der Hülse von Figur 1 in vergrößerter Ansicht;

Figur 3 den Ausschnitt von Figur 2 in einer alternativen Ausführungsform;

Figur 4 eine Hülse 1 zum Absaugen von Bruchstücken bei der operativen Behandlung von grauem Star gemäß einer weiteren Ausführungsform der Erfindung;

Figur 5    ein Flussdiagramm eines Verfahrens zum Bearbeiten eines Hülsenrohlings gemäß einer Ausführungsform der Erfindung; und

Figur 6    ein Flussdiagramm eines Verfahrens zur Herstellung einer Hülse zum Absaugen von Bruchstücken bei der operativen Behandlung von grauem Star gemäß einer Ausführungsform der Erfindung.

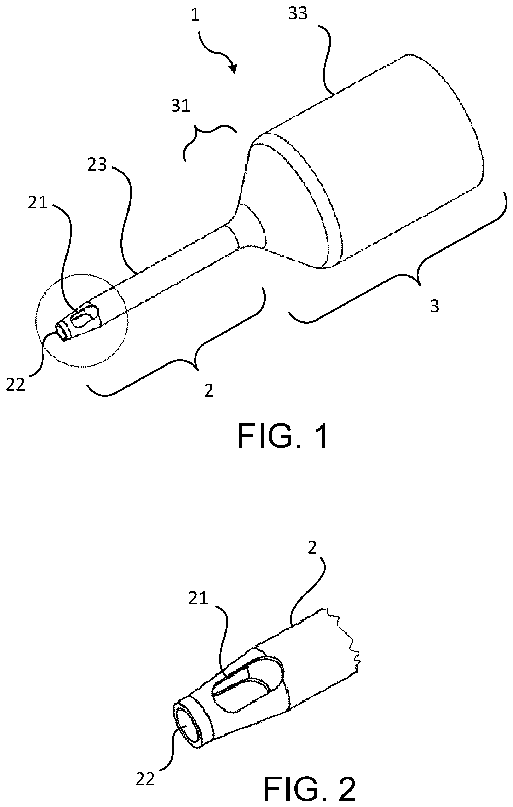

**Figur 1** zeigt eine Hülse 1 zum Absaugen von Bruchstücken bei der operativen Behandlung von grauem Star gemäß einer Ausführungsform der Erfindung. Die Hülse 1 kann beispielsweise eine Silikonhülse, insbesondere eine spritzgegossene Silikonhülse, sein. Die Hülse 1 umfasst einen operativen Bereich 2 und einen Anschlussbereich 3, der an den operativen Bereich 2 anschließt. Vorzugsweise können der Anschlussbereich 3 und der operative Bereich 2 ein gemeinsamer einteiliger Körper sein. Der operative Bereich 2 umfasst einen ersten rohrartigen Hohlabschnitt 23, durch den abgesaugte Bruchstücke der Linse während der Behandlung transportiert werden können. Der operative Bereich 2 wurde im vorderen Abschnitt des ersten rohrartigen Hohlabschnitts 23 mit einem Laserstrahl eines Femtosekundenlasers bearbeitet, sodass durch die Laserbearbeitung, nämlich durch Laserschneiden, Material von dem operativen Bereich 2 entfernt worden ist, um ein seitliches Loch 21 zu erzeugen. Zudem wurde ein Ende 22 des operativen Bereichs 2, ebenfalls durch einen Laserstrahl, insbesondere durch ein Laserschneiden, abgelängt. Zum Laserschneiden eines Lochs, wie auch zum Ablängen wird erfindungsgemäß ein Laserstrahl mit einer Spotgröße von 2 bis 25 Mikrometern, besonders bevorzugt 8 bis 20 Mikrometern, und mit einer Pulsleistung von zumindest 25 Watt, besonders bevorzugt zumindest 35 Watt, verwendet. Es hat sich herausgestellt, dass mit diesen Werten Rückstände durch einen Abbrand an der Hülse in der Umgebung der Laserbearbeitung deutlich reduziert werden können bzw. sogar im Wesentlichen komplett vermieden werden können. Der Anschlussbereich der Hülse umfasst einen zweiten rohrartigen Hohlabschnitt 33, der einen größeren Querschnittsdurchmesser aufweist als der erste rohrartige Hohlabschnitt 23 des operativen Bereichs 2. Insbesondere weist der zweite rohrartige Hohlabschnitt 33 in diesem Beispiel einen ungefähr fünfmal so großen äußeren Querschnittsdurchmesser auf wie der erste rohrartige Hohlabschnitt 34. Der erste rohrartige Hohlabschnitt 23 und der zweite rohrartige Hohlabschnitt 33 sind im Wesentlichen konzentrisch zueinander angeordnet. Der Anschlussbereich 3 weist weiterhin einen Übergangsbereich 31 auf, der zwischen dem ersten rohrartigen Hohlabschnitt 23 und dem zweiten rohrartigen Hohlabschnitt 33 ist, sodass der erste rohrartige Hohlabschnitt 23 mittels des Übergangsbereichs 31 in den zweiten rohrartigen Hohlabschnitt 33 übergeht. Dabei geht insbesondere auch ein innerer Hohlbereich des ersten rohrartigen Hohlabschnitt 23 in einen inneren Hohlbereich des zweiten rohrartigen Hohlabschnitt 33 über. Der Übergangsbereich 31 ist im Wesentlichen konzentrisch zu dem ersten rohrartigen Hohlabschnitt 23 und zu dem zweiten rohrartigen Hohlabschnitt 33.

**Figur 2** zeigt den mit einem Kreis markierten Ausschnitt der Hülse 1 von Figur 1 in vergrößerter Ansicht. Zu sehen ist der vordere Abschnitt des ersten rohrartigen Hohlabschnitts 23 in dem ein seitliches Loch 21 ist. Das seitliche Loch 21 weist in dieser Ausführungsform eine längliche Erstreckung parallel zu der Längserstreckung des ersten rohrartigen Hohlabschnitts 23 auf. Das seitliche Loch ist in dieser Ausführungsform ein Querloch bzw. eine Querbohrung, das/die sich komplett durch den ersten rohrartigen Hohlabschnitts 23 erstreckt, sodass der erste rohrartige Hohlabschnitt an zwei gegenüberliegenden seitlichen Seiten eine Öffnung aufweist. Die Ecken des seitlichen Lochs 21 sind abgerundet. Es ist zudem eine vordere Öffnung am Ende 22 des operativen Bereichs 2 zu erkennen.

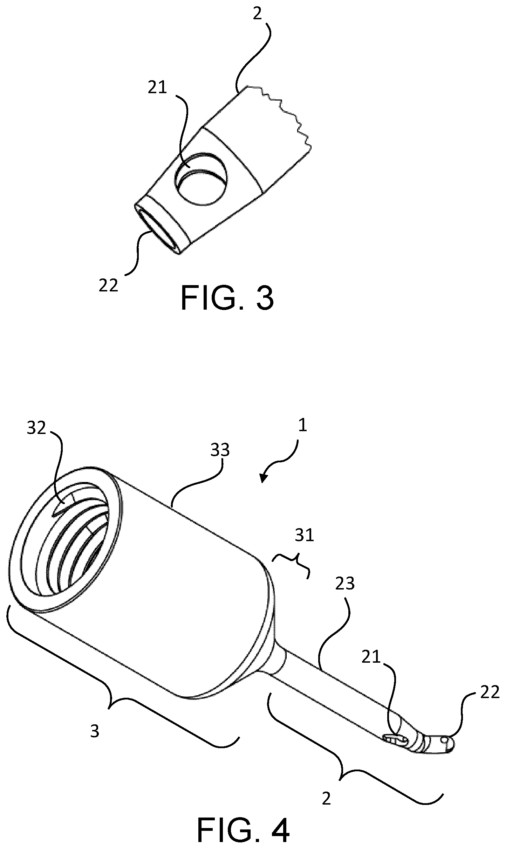

**Figur 3** zeigt den Ausschnitt von Figur 2 in einer alternativen Ausführungsform. In dieser Ausführungsform ist das seitliche Loch 21 im Wesentlichen als runde Öffnung ausgestaltet. Vorteilhafterweise kann mit dem erfindungsgemäßen Verfahren eine Anpassung der geometrischen Eigenschaften des vorgesehenen seitlichen Lochs 21 mit relativ geringem Aufwand ermöglicht werden. Beispielsweise kann eine Anpassung des Lochs durch eine Programmanpassung in einem Programm, das den Laserstrahl steuert realisiert werden.

**Figur 4** zeigt eine Hülse 1 zum Absaugen von Bruchstücken bei der operativen Behandlung von grauem Star gemäß einer weiteren Ausführungsform der Erfindung. Dieser Ausführungsform unterscheidet sich von derjenigen in Figur 1 durch das Ende 22 des rohrartigen Hohlabschnitts 23. Das Ende 22 ist in dieser Ausführungsform abgeknickt bzw. weißt einen Winkel auf. Diese Ausführungsform kann für manche operative Anforderungen vorteilhaft sein. In dieser Darstellung ist zudem ein Innengewinde 32 zu sehen, welches in dem Anschlussbereich vorgesehen ist. Optional kann auch die Ausführungsform, die in Figur 1 gezeigt ist ein solches oder ein ähnliches Innengewinde 32 aufweisen. Das Innengewinde kann beispielsweise zum Einschrauben eines Anschlussschlauchs und/oder -kabels vorgesehen sein.

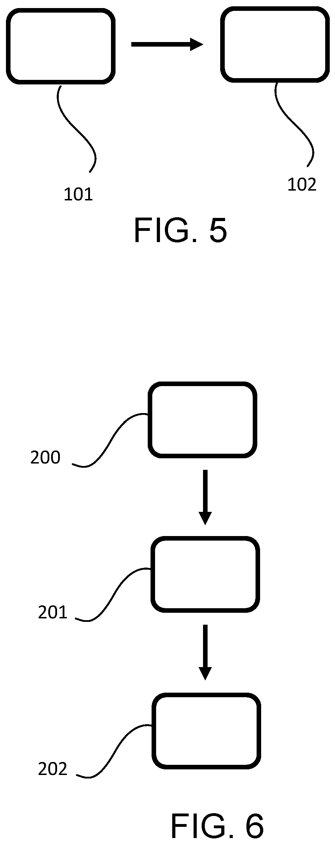

**Figur 5** zeigt ein Flussdiagramm eines Verfahrens zum Bearbeiten eines Hülsenrohlings gemäß einer Ausführungsform der Erfindung. In einem ersten Schritt 101 wird ein Hülsenrohling bereitgestellt. Der Hülsenrohling kann vorzugsweise ein Silikonhülsenrohling sein. In einem weiteren Schritt 102 wird ein Laserstrahls auf zumindest einen vorbestimmten Fokuspunkt auf dem Hülsenrohling fokussiert, sodass von dem Hülsenrohling an dem zumindest einen Fokuspunkt mit Hilfe der Laserenergie Material entfernt wird. Der Hülsenrohling kann insbesondere mit Hilfe der Laserenergie abgelängt und/oder mit einer Ausnehmung versehen werden.

Die Ausnehmung kann insbesondere ein seitliches Loch 21 sein. Dabei wird der Laserstrahl von einem Ultrakurzpulslaser, insbesondere Femtosekundenlaser, erzeugt, wobei der Laserstrahl eine Spotgröße von 2 bis 25 Mikrometern, vorzugsweise 5 bis 22 Mikrometern, besonders bevorzugt 8 bis 20 Mikrometern, und eine Pulsleistung von zumindest 25 Watt, vorzugsweise zumindest 30 Watt, besonders bevorzugt zumindest 35 Watt, aufweist. Vorzugsweise kann der Laserstrahl eine Pulsenergie von zumindest 120 Mikrojoule, vorzugsweise zumindest 150 Mikrojoule, besonders bevorzugt zumindest 180 Mikrojoule, aufweisen. Vorzugsweise kann der Laserstrahl eine Wellenlänge von 200 bis 500 nm, vorzugsweise 300 bis 400 nm, ganz bevorzugt 330 bis 380 nm, aufweisen. Um die Wellenlänge des Laserstrahls einzustellen kann optional ein Modul zur Frequenzvervielfachung verwendet werden. Beispielsweise kann der verwendete Ultrakurzpulslaser dazu ausgestaltet sein, Laserlicht im Infrarotbereich, beispielsweise bevorzugt zwischen 1000 und 1200 nm, zu erzeugen. Dabei kann ein Modul zur Frequenzverdreifachung eingesetzt werde, um die Wellenlänge des Laserstrahls, der auf den Fokuspunkt trifft, auf einen gewünschten Wellenlängenwert, insbesondere vorzugsweise im Bereich von 300 nm bis 400 nm, einzustellen. Tests bei einer Wellenlänge von 343 nm haben ergeben, dass damit sehr gute Ergebnisse erzielt werden können bei einer Bearbeitungsdauer von 3,3 Sekunden pro Loch, wobei auch eine Bearbeitungsdauer von 1,54 Sekunden pro Loch mit guten Ergebnissen erzielt werden konnte. Es kann vorzugsweise optional vorgesehen sein, während der Bearbeitung der Hülse mit dem Laserstrahl eine Laserrauch-Absaugvorrichtung einzusetzen, um einen entstehenden Laserabbrand abzusaugen. Damit kann vorteilhafterweise noch verbleibender Laserabbrand reduziert werden.

**Figur 6** zeigt ein Flussdiagramm eines Verfahrens zur Herstellung einer Hülse 1 zum Absaugen von Bruchstücken bei der operativen Behandlung von grauem Star gemäß einer Ausführungsform der Erfindung. In einem ersten Schritt 200 wird ein Hülsenrohling spritzgegossen. Insbesondere kann der Hülsenrohling aus einem Silikonmaterial sein und die mit dem Verfahren hergestellte Hülse kann entsprechend eine Silikonhülse sein. Die folgenden Schritte 201, 202 können optional unmittelbar in den ersten Schritt 200 integriert sein bzw. an diesen anschließen. In einem weiteren Schritt 201 wird der Hülsenrohling zur Laserbearbeitung bereitgestellt. In einem noch weiteren Schritt 202 wird ein Laserstrahls auf zumindest einen vorbestimmten Fokuspunkt auf dem Hülsenrohling fokussiert, sodass von dem Hülsenrohling an dem zumindest einen Fokuspunkt mit Hilfe der Laserenergie Material entfernt wird. Die weiteren Schritte 201, 202 können im Wesentlichen den Schritten 101, 102 des mit Bezug zu Figur 5 beschriebenen Verfahrens entsprechen.

Bezugszeichenliste:

| | |
|---|---|
| 1 | - Hülse |
| 2 | - operativer Bereich |
| 3 | - Anschlussbereich |
| 21 | - seitliches Loch |
| 22 | - Ende des operativen Bereichs |
| 23 | - erster rohrartiger Hohlabschnitt |
| 31 | - Übergangsbereich |
| 32 | - Innengewinde |
| 33 | - zweiter rohrartiger Hohlabschnitt |

**Patentansprüche**

1. Hülse (1), insbesondere Silikonhülse, zum Absaugen von Bruchstücken bei der operativen Behandlung von grauem Star,

   wobei die Hülse (1) einen operativen Bereich (2) und einen Anschlussbereich (3), der an den operativen Bereich (2) anschließt, umfasst, wobei der operative Bereich (2) zumindest abschnittsweise mit einem Laserstrahl eines Ultrakurzpulslasers, insbesondere Femtosekundenlasers, laserbearbeitet ist, sodass durch die Laserbearbeitung Material von dem operativen Bereich (2) entfernt worden ist, wobei der Laserstrahl bei der Laserbearbeitung eine Spotgröße von 2 bis 25 Mikrometern, vorzugsweise 5 bis 22 Mikrometern, besonders bevorzugt 8 bis 20 Mikrometern und eine Pulsleistung von zumindest 25 Watt, vorzugsweise zumindest 30 Watt, besonders bevorzugt zumindest 35 Watt, aufweist bzw. aufgewiesen hat.

2. Hülse (1) gemäß Anspruch 1,

   wobei der operative Bereich (2) ein Loch bzw. eine Bohrung, insbesondere seitliches Loch bzw. seitliche Bohrung (21), umfasst, die während der Laserbearbeitung durch Laserschneiden mit dem Laserstrahl erzeugt worden ist und/oder, wobei ein Ende (22) des operativen Bereichs während der Laserbearbeitung mit dem Laserstrahl abgelängt worden ist.

3. Hülse (1) gemäß einem der vorhergehenden Ansprüche, wobei die Hülse so hergestellt ist, dass während der Bearbeitung der Hülse (1) mit dem Laserstrahl eine Laserrauch-Absaugvorrichtung eingesetzt wurde, um einen entstehenden Laserabbrand abzusaugen.

**4.** Hülse (1) gemäß einem der vorhergehenden Ansprüche,
wobei der operative Bereich (2) einen ersten rohrartiger Hohlabschnitt (23) mit zumindest einem seitlichen Loch (21) umfasst, wobei das seitliche Loch (21) durch Laserschneiden mit dem Laserstrahl erzeugt worden ist.

**5.** Hülse (1) gemäß Anspruch 4,

wobei der Anschlussbereich (2) der Hülse (1) einen zweiten rohrartigen Hohlabschnitt (33) umfasst,
wobei der zweite rohrartige Hohlabschnitt (33) einen größeren Querschnittsdurchmesser aufweist als der erste rohrartige Hohlabschnitt (23), insbesondere einen zumindest zweimal so großen Querschnittsdurchmesser, vorzugsweise zumindest dreimal so großen Querschnittsdurchmesser.

**6.** Hülse (1) gemäß Anspruch 5,
wobei ein Verhältnis des Außendurchmessers des ersten rohrartigen Hohlabschnitts (23) zu einem Innendurchmesser des ersten rohrartigen Hohlabschnitts (23) geringer als ein Verhältnis des Außendurchmessers des zweiten rohrartigen Hohlabschnitts (33) zu einem Innendurchmesser des zweiten rohrartigen Hohlabschnitts (33).

**7.** Hülse (1) gemäß Anspruch 5 oder 6,
wobei der Anschlussbereich (2) einen Übergangsbereich zwischen dem ersten rohrartigen Hohlabschnitt (23) und dem zweiten rohrartigen Hohlabschnitt (33) aufweist, sodass der erste rohrartige Hohlabschnitt (23) in den zweiten rohrartigen Hohlabschnitt (33) übergeht, wobei insbesondere ein innerer Hohlbereich des ersten rohrartigen Hohlabschnitts (23) in einen inneren Hohlbereich des zweiten rohrartigen Hohlabschnitts (33) übergeht.

**8.** Hülse (1) gemäß einem der vorhergehenden Ansprüche,
wobei der Anschlussbereich (3) und der operative Bereich (2) ein gemeinsamer einteiliger Körper sind.

**9.** Hülse (1) gemäß einem der vorhergehenden Ansprüche,
wobei die Hülse (1) spritzgegossen ist.

**10.** Hülse (1) gemäß einem der vorhergehenden Ansprüche,
wobei die Hülse (1) aus einem transparenten Silikonmaterial gefertigt, insbesondere spritzgegossen, ist

**11.** Verwendung eines Ultrakurzpulslasers, insbesondere Femtosekundenlasers, zum Entfernen von Material durch Laserbearbeitung von einem Hülsenrohling, insbesondere Silikonhülsenrohling, bei der Herstellung einer Hülse (1) zum Absaugen von Bruchstücken bei der operativen Behandlung von grauem Star,
wobei der verwendete Ultrakurzpulslaser einen Laserstrahl mit einer Spotgröße von 2 bis 25 Mikrometern, vorzugsweise 5 bis 22 Mikrometern, besonders bevorzugt 8 bis 20 Mikrometern und einer Pulsleistung von zumindest 25 Watt, vorzugsweise zumindest 30 Watt, besonders bevorzugt zumindest 35 Watt, erzeugt.

**12.** Verfahren zum Bearbeiten eines Hülsenrohlings, insbesondere Silikonhülsenrohlings, zum Absaugen von Bruchstücken bei der operativen Behandlung von grauem Star, um Material von dem Hülsenrohling zu entfernen, insbesondere um den Hülsenrohling abzulängen und/oder mit zumindest einer Ausnehmung zu versehen, insbesondere zur Herstellung einer Hülse (1) gemäß einem der vorhergehenden Ansprüche, wobei das Verfahren umfasst:

- Bereitstellen des Hülsenrohlings
- Fokussieren eines Laserstrahls auf zumindest einen vorbestimmten Fokuspunkt auf dem Hülsenrohling, sodass von dem Hülsenrohling an dem zumindest einen Fokuspunkt mit Hilfe der Laserenergie Material entfernt wird, wobei der Hülsenrohling insbesondere mit Hilfe der Laserenergie abgelängt und/oder mit einer Ausnehmung versehen wird; wobei der Laserstrahl von einem Ultrakurzpulslaser, insbesondere Femtosekundenlaser, erzeugt wird, wobei der Laserstrahl eine Spotgröße von 2 bis 25 Mikrometern, vorzugsweise 5 bis 22 Mikrometern, besonders bevorzugt 8 bis 20 Mikrometern, und eine Pulsleistung von zumindest 25 Watt, vorzugsweise zumindest 30 Watt, besonders bevorzugt zumindest 35 Watt, aufweist.

**13.** Verfahren gemäß Anspruch 12,

wobei der Ultrakurzpulslaser dazu ausgestaltet ist, Laserlicht im Infrarotbereich, insbesondere mit einer Wellenlänge von zumindest 900 nm, vorzugsweise zwischen 900 und 1500 nm, ganz bevorzugt zwischen 1000 und 1200 nm, zu erzeugen,
wobei ein Modul zur Frequenzvervielfachung, insbesondere Frequenzverdreifachung, eingesetzt wird, um die Wellenlänge des Laserstrahls, der auf den zumindest einen Fokuspunkt trifft, auf einen geringeren Wellenlängenwert einzustellen.

**14.** Verfahren gemäß Anspruch 12 oder 13,
wobei der Laserstrahl eine Wellenlänge von 200 bis 500 nm, vorzugsweise 300 bis 400 nm, ganz bevorzugt 330 bis 380 nm aufweist.

**15.** Verfahren zur Herstellung einer Hülse, insbesondere Silikonhülse, zum Absaugen von Bruchstücken bei der operativen Behandlung von grauem Star, umfassend die folgenden Schritte:

- Spritzgießen eines Hülsenrohlings, insbesondere aus einem Silikonmaterial, in einem Spritzgießverfahren,
- Bearbeiten des spritzgegossenen Hülsenrohlings mit einem Verfahren gemäß einem der Ansprüche 12 bis 14, um die Hülse (1) abzulängen und/oder mit zumindest einer Ausnehmung zu versehen.

1

33

31

23

21

22

2

3

# FIG. 1

2

21

22

# FIG. 2

FIG. 3

FIG. 4

101

102

## FIG. 5

200

201

202

## FIG. 6

| | Europäisches Patentamt European Patent Office Office européen des brevets | EUROPÄISCHER RECHERCHENBERICHT | Nummer der Anmeldung EP 25 20 3544 |
|---|---|---|---|

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2020/306087 A1 (GARCIA JOSE L [US] ET AL) 1. Oktober 2020 (2020-10-01) * Absatz [0026] - Absatz [0048]; Abbildungen 3,4,5a * ----- | 1-15 | INV. A61M1/00 A61F9/007 A61M3/02 B23K26/0622 B23K26/382 |
| A | US 2006/285071 A1 (ERICKSON PAUL M [US] ET AL) 21. Dezember 2006 (2006-12-21) * Absatz [0012]; Anspruch 6 * ----- | 1-15 | ADD. A61F9/008 |
| A | WO 2024/043220 A1 (YOKOWO SEISAKUSHO KK [JP]) 29. Februar 2024 (2024-02-29) * Absatz [0031] - Absatz [0032]; Abbildung 9 * ----- | 1-15 | |
| A | US 6 428 501 B1 (REYNARD MICHAEL [US]) 6. August 2002 (2002-08-06) * Spalte 4, Zeile 20; Anspruch 4; Abbildung 1 * ----- | 1-15 | |
| A | Fxstageadmin: "Silicon Tube Processing By Laser Advanced Optowave", , 23. Juli 2024 (2024-07-23), XP093362918, Gefunden im Internet: URL:https://web.archive.org/web/2024072319 5114/https://www.a-optowave.com/news/laser -processing-of-silicone-tube/ [gefunden am 2026-02-05] * das ganze Dokument * ----- | 1-15 | RECHERCHIERTE SACHGEBIETE (IPC) A61M B23K A61F |
| A | US 4 983 160 A (STEPPE DENNIS L [US] ET AL) 8. Januar 1991 (1991-01-08) * das ganze Dokument * ----- | 1-15 | |
| A | JP 2003 156667 A (SEIKO EPSON CORP) 30. Mai 2003 (2003-05-30) * das ganze Dokument * ----- | 1-15 | |

-/--

Der vorliegende Recherchenbericht wurde für alle Patentansprüche erstellt

| Recherchenort | Abschlußdatum der Recherche | Prüfer |
|---|---|---|
| Den Haag | 6. Februar 2026 | Weiss-Schaber, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...............................................................
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

**Nummer der Anmeldung**

EP 25 20 3544

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | EP 4 101 486 A1 (APTAR RADOLFZELL GMBH [DE]) 14. Dezember 2022 (2022-12-14) * Absatz [0041] - Absatz [0042] * ----- | 1-15 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| Den Haag | 6. Februar 2026 | Weiss-Schaber, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 20 3544

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-02-2026

| Im Recherchenbericht angeführtes Patentdokument | Datum der Veröffentlichung | Mitglied(er) der Patentfamilie | Datum der Veröffentlichung |
|---|---|---|---|
| US 2020306087 A1 | 01-10-2020 | AU 2020255351 A1 | 03-12-2020 |
| | | CA 3100523 A1 | 08-10-2020 |
| | | EP 3946184 A1 | 09-02-2022 |
| | | US 2020306087 A1 | 01-10-2020 |
| | | WO 2020201884 A1 | 08-10-2020 |
| US 2006285071 A1 | 21-12-2006 | CA 2612023 A1 | 04-01-2007 |
| | | CN 101198434 A | 11-06-2008 |
| | | EP 1893382 A1 | 05-03-2008 |
| | | TW 200704466 A | 01-02-2007 |
| | | US 2006285071 A1 | 21-12-2006 |
| | | WO 2007002231 A1 | 04-01-2007 |
| WO 2024043220 A1 | 29-02-2024 | JP WO2024043220 A1 | 29-02-2024 |
| | | TW 202411001 A | 16-03-2024 |
| | | WO 2024043220 A1 | 29-02-2024 |
| US 6428501 B1 | 06-08-2002 | KEINE | |
| US 4983160 A | 08-01-1991 | KEINE | |
| JP 2003156667 A | 30-05-2003 | KEINE | |
| EP 4101486 A1 | 14-12-2022 | CN 117500550 A | 02-02-2024 |
| | | EP 4101486 A1 | 14-12-2022 |
| | | US 2024269371 A1 | 15-08-2024 |
| | | WO 2022258462 A1 | 15-12-2022 |

Für nähere Einzelheiten zu diesem Anhang : siehe Amtsblatt des Europäischen Patentamts, Nr.12/82